(19) Europäisches Patentamt
European Patent Office
Office européen des brevets

(11) **EP 2 123 327 B1**

(12) **EUROPÄISCHE PATENTSCHRIFT**

(45) Veröffentlichungstag und Bekanntmachung des Hinweises auf die Patenterteilung:
**08.05.2013 Patentblatt 2013/19**

(51) Int Cl.:
***A61N 5/10*** *(2006.01)*

(21) Anmeldenummer: **09006963.4**

(22) Anmeldetag: **25.05.2009**

(54) **Vorrichtung und Verfahren zur Markierung eines Bestrahlungsfeldes auf der Oberfläche eines Patientenkörpers**

Device and method for marking an irradiation field on the surface of a patient's body

Dispositif et procédé de marquage d'un champ de rayonnement sur la surface du corps d'un patient

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **24.05.2008 DE 102008025014**

(43) Veröffentlichungstag der Anmeldung:
**25.11.2009 Patentblatt 2009/48**

(73) Patentinhaber: **LAP GmbH Laser Applikationen D-21337 Lüneburg (DE)**

(72) Erfinder:
• **Kindlein, Johann**
**21339 Lüneburg (DE)**
• **Thurn, Tim**
**21335 Lüneburg (DE)**

(74) Vertreter: **Hauck Patent- und Rechtsanwälte Neuer Wall 50**
**20354 Hamburg (DE)**

(56) Entgegenhaltungen:
**EP-A- 0 581 704       EP-A- 1 640 922**
**WO-A-02/09588       DE-A1- 19 524 951**
**US-A1- 2008 025 638**

EP 2 123 327 B1

## Beschreibung

Die vorliegende Erfindung betrifft eine Vorrichtung und ein Verfahren zur Markierung eines anhand eines virtuellen 3D-Modells eines Patientenkörpers erstellten Bestrahlungsfeldes auf der Oberfläche des Patientenkörpers. Solche Bestrahlungsfelder stellen Soll-Schnittfelder eines insbesondere zur Krebsbehandlung genutzten Therapiestrahls meist aus ionisierender Strahlung mit der Oberfläche eines Patientenkörpers dar. Dabei werden üblicherweise mehrere Strahlen aus unterschiedlichen Richtungen auf den zu behandelnden Körper gerichtet, so dass sie sich in einem Isozentrum schneiden. Dort wirkt die summierte Strahlendosis und die Beeinträchtigung umliegenden Gewebes wird minimiert. Als erster Schritt einer Strahlentherapie wird üblicherweise eine Tomographie-Aufnahme, beispielsweise Computer-Tomographie (CT), einer interessierenden Körperregion des auf einer Lagerungshilfe, wie einem Behandlungstisch, unter Einsatz spezieller Lagerungs- und Fixierungshilfen gelagerten und fixierten Patienten erstellt. Dabei wird ein Referenzpunkt für die Tomographieaufnahme durch Markierung der Hautoberfläche des Patienten mit dem Lasersystem in der Referenzposition, meist der Nullposition, festgelegt. Anhand der Tomographie-Aufnahme wird ein 3D-Modell der interessierenden Region des Patientenkörpers erstellt und der zu behandelnde Tumor lokalisiert. Im Rahmen der Bestrahlungsplanung werden die Zielvolumina konturiert, die Bestrahlungsdosis berechnet und dabei insbesondere die Anzahl und Lage der Bestrahlungsfelder bestimmt, so dass der Tumor wie gewünscht bestrahlt wird.

Die anschließende Simulation ist die Übertragung der Koordinaten (Lage und Form) der berechneten Bestrahlungsfelder aus dem berechneten Modell auf den realen Patienten und umfasst üblicherweise eine Markierung der Behandlungsfelder auf der Haut des Patienten, beispielsweise mit einem Stift. Vor der tatsächlichen Bestrahlung kann mittels eines den Behandlungsstrahl darstellenden Lichtfelds des Bestrahlungsgeräts die korrekte Lage der Bestrahlungsfelder überprüft werden. Der Markierungsprozess ist die Übertragung von Koordinaten aus dem statischen 3D-Patientenmodell auf den lebenden Patienten. Da es sich dabei um lebende Menschen mit weichen Körperstrukturen, und nicht um starre Körper handelt, ist eine genaue und reproduzierbare Positionierung besonders bei sehr schwer erkrankten und adipösen Patienten kompliziert.

Zur Markierung werden Laser-Markierungssysteme verwendet. Ein bekanntes Laser-Markierungssystem ist in DE 44 21 315 A1 oder DE 195 24 951 A1 beschrieben und besteht aus insgesamt fünf motorisch beweglichen Lasern, die im Raum des Tomographiegeräts montiert sind. Zwei in der Höhe verstellbare Laser, die jeweils eine horizontale Linie entlang des den Patienten während der Tomographie aufnehmenden Tisches projizieren, befinden sich rechts und links des Tisches. Die übrigen drei Laser sind in einer Platte oder Halterung montiert, die sich über dem Tisch befindet. Ein Laser ist quer zur Tisch-Längsrichtung verschiebbar und projiziert eine Linie entlang des Tisches. Zwei der Laser in der Platte oder Halterung sind miteinander gekoppelt und projizieren eine gemeinsame Linie quer zur Längsachse des Tisches. Dabei werden zwei Laser gekoppelt, damit durch die seitliche Anordnung längs der Patientenachse auf dem Tisch auch noch solche Koordinaten auf der Haut dargestellt werden können, die andernfalls unterhalb des Querdurchmessers des Patienten durch den Patienten verschattet würden. Ein solches Lasersystem wird von der Anmelderin unter dem Namen "Dorado CT 4" vertrieben.

Zur Markierung der berechneten Bestrahlungsfelder mit dem bekannten Laser-Markierungssystem erfolgt zunächst eine Lagerung und Fixierung des Patienten in einer Position, die möglichst genau mit der Position des Patienten bei der Erstellung des Tomographie-Scans übereinstimmt. Um die Positionierung des Patienten zu überprüfen, wird nach der Einstellung der Referenzebene (Laser in der Referenzposition, meist Nullposition) eine erneute Tomographie (z.B. CT) durchgeführt. Sollten bei dieser Lagekontrolle größere Abweichungen festgestellt werden, so ist die Lagerung und Fixierung des Patienten zu wiederholen bzw. zu korrigieren. Dazu kann der den Patient aufnehmende Behandlungstisch entsprechend neu positioniert werden. Nach Abschluss der Lagekontrolle können die Markierungen auf der Patientenhaut mit Hilfe des Laser-Markierungssystems durchgeführt werden. Dazu werden die zuvor berechneten Koordinaten der gewünschten Bestrahlungsfelder dem Laser-Markierungssystem zur Verfügung gestellt. Die Koordinaten können dann sukzessive mittels des Laser-Markierungssystems angefahren und somit für einen Bearbeiter angezeigt und dann beispielsweise mittels eines Stifts manuell auf der Haut markiert werden.

Bei der aus dem Stand der Technik bekannten Vorgehensweise ist nachteilig, dass die Markierung aufgrund der Lagekontrolle mittels eines erneuten Tomographie-Scans in dem Raum der Tomographieeinrichtung erfolgen muss. Dies beansprucht wertvolle Zeit in diesem Raum. Außerdem ist mit der erneuten Lagekontrolle mittels Tomographie-Scan eine Strahlenbelastung für den Patienten verbunden.

Des weiteren wird in der EP 1 640 922 A2 ein System mit einem Linearbeschleuniger zur Positionierung eines Patienten offenbart. Dabei wird eine Transformation zum Angleichen einer erzeugten Modelloberfläche an eine gespeicherte Oberfläche durchgeführt.

Ausgehend von dem erläuterten Stand der Technik liegt der Erfindung die Aufgabe zugrunde, eine Vorrichtung und ein Verfahren der eingangs genannten Art bereitzustellen, mit denen die Markierung in einfacher und kostengünstiger Weise möglich ist, wobei die gesundheitliche Beeinträchtigung des Patienten minimiert wird.

Diese Aufgabe wird gemäß der Erfindung durch die Gegenstände der unabhängigen Ansprüche 1 und 14 gelöst. Vorteilhafte Ausgestaltungen finden sich in den abhängigen Ansprüchen sowie der Beschreibung und den Figuren.

Die Erfindung löst die Aufgabe zum einen durch eine Vorrichtung zur Markierung eines anhand eines virtuellen 3D-Modells eines Patientenkörpers erstellten Bestrahlungsfeldes auf der Oberfläche des Patientenkörpers, mit einem Lasersystem zur Ermittlung der Koordinaten mindestens zweier Referenzpunkte auf der Oberfläche des Patientenkörpers in einem der Vorrichtung zugeordneten lokalen Koordinatensystem, mit einer Auswerte- und Steuereinrichtung, die dazu ausgebildet ist, aus den Koordinaten der Referenzpunkte in dem lokalen Koordinatensystem und aus Koordinaten der Referenzpunkte in einem virtuellen Koordinatensystem des virtuellen 3D-Modells des Patientenkörpers eine Transformationsmatrix zur Transformation beliebiger Koordinaten aus dem virtuellen Koordinatensystem in das lokale Koordinatensystem zu ermitteln, und wobei die Auswerte- und Steuereinrichtung weiter dazu ausgebildet ist, mit der Transformationsmatrix Koordinaten aus dem virtuellen Koordinatensystem in das lokale Koordinatensystem zu transformieren und die transformierten Koordinaten dem Lasersystem zur Verfügung zu stellen.

Zum anderen löst die Erfindung die Aufgabe durch ein Verfahren zur Markierung eines anhand eines virtuellen 3D-Modells eines Patientenkörpers erstellten Bestrahlungsfeldes auf der Oberfläche des Patientenkörpers, bei dem die Koordinaten mindestens zweier Referenzpunkte auf der Oberfläche des Patientenkörpers in einem der Vorrichtung zugeordneten lokalen Koordinatensystem ermittelt werden, bei dem aus den Koordinaten der Referenzpunkte in dem lokalen Koordinatensystem und aus Koordinaten der Referenzpunkte in einem virtuellen Koordinatensystem des virtuellen 3D-Modells des Patientenkörpers eine Transformationsmatrix zur Transformation beliebiger Koordinaten aus dem virtuellen Koordinatensystem in das lokale Koordinatensystem ermittelt wird, und bei dem mit der Transformationsmatrix Koordinaten aus dem virtuellen Koordinatensystem in das lokale Koordinatensystem transformiert werden und für die Markierung zur Verfügung gestellt werden. Das Lasersystem kann insbesondere ein Laser-Markierungssystem sein.

Die Auswerte- und Steuereinrichtung kann dazu ausgebildet sein, die Koordinaten zumindest des Bestrahlungsisozentrums aus dem virtuellen Koordinatensystem in das lokale Koordinatensystem zu transformieren und die transformierten Koordinaten dem Lasersystem zur Verfügung zu stellen. Als Bestrahlungsisozentrum wird dabei das Isozentrum eines im Rahmen einer Krebsbehandlung zu bestrahlenden Tumors bezeichnet. Um dieses Isozentrum dreht sich ein für die Bestrahlung eingesetztes Bestrahlungsgerät (z.B. ein Linearbeschleuniger) während der Bestrahlung. Dieses im Inneren des Patienten liegende Isozentrum wird üblicherweise durch drei Punkte auf der Haut des Patienten markiert.

Diese Punkte dienen dann zur Positionierung des Patienten am Bestrahlungsgerät. Werden als Referenzpunkte beispielsweise die Koordinaten dieser Punkte ermittelt, kann auf dieser Basis die Transformationsmatrix bestimmt werden. Die Auswerte- und Steuereinrichtung kann weiter dazu ausgebildet sein, mit der Transformationsmatrix weitere Koordinaten aus dem virtuellen Koordinatensystem in das lokale Koordinatensystem zu transformieren und die transformierten Koordinaten dem Lasersystem zur Verfügung zu stellen. So können beispielsweise die Koordinaten des Umrisses und/oder der Fläche und/oder der Form des Bestrahlungsfeldes aus dem virtuellen Koordinatensystem in das lokale Koordinatensystem transformiert werden und für die Markierung zur Verfügung gestellt werden. Das Bestrahlungsfeld kann ein für ein beispielsweise auf Grundlage einer Tomographie-Aufnahme (z.B. CT-Aufnahme) erstelltes virtuelles 3D-Modell eines Patienten berechnetes Soll-Schnittfeld eines zur Krebsbehandlung eingesetzten Therapiestrahls (z.B. ionisierende Strahlung) mit der Haut des Patienten darstellen. Natürlich können dabei mehrere Bestrahlungsfelder zur Markierung vorgesehen sein. Das 3D-Modell kann eine interessierende Region des Patientenkörpers oder auch den gesamten Körper darstellen. Die Koordinaten sind insbesondere dreidimensionale Koordinaten.

Die Grundidee der Erfindung liegt darin, im Gegensatz zum Stand der Technik den Patienten in dem das Lasersystem aufweisenden Markierungsraum nicht über eine Verlagerung des Behandlungstisches sowie des Patienten so zu positionieren, dass er wieder dieselbe Position einnimmt, wie bei der Tomographieaufnahme für die Bestrahlungsplanung. Stattdessen werden erfindungsgemäß die Koordinaten der zu markierenden Bestrahlungsfelder an die (neue) Position des Patienten in dem Markierungsraum angepasst. Der Patient selbst kann also in weiten Grenzen beliebig positioniert werden und muss aufgrund der Koordinatentransformation nicht aufwendig in seiner Positionierung angepasst werden. In dem Markierungsraum kann der Patient mit einer Lagerungshilfe gelagert sein. Diese muss aus den genannten Gründen jedoch nicht unbedingt beweglich sein

Die Koordinaten der in dem Markierungsraum angesteuerten Referenzpunkte sind nach der Ansteuerung mit den Laserlinien in dem lokalen Koordinatensystem der Vorrichtung bekannt. Außerdem sind die Koordinaten der Referenzpunkte auch in dem virtuellen Koordinatensystem des im Rahmen der Bestrahlungsplanung erstellten virtuellen 3D-Modells des Patientenkörpers bekannt. Auf dieser Grundlage kann die Transformationsmatrix bestimmt werden. Die Transformationsmatrix wird dabei anhand eines Vergleichs der Referenzpunkte, und insbesondere der Transformation der Referenzpunkte aus dem virtuellen Koordinatensystem in das lokale Koordinatensystem, ermittelt. Der Patient dient mittels der Referenzpunkte als Übergang zwischen dem im Rahmen der Bestrahlungsplanung erstellten virtuellen 3D-Modell seines Körpers und seinem Körper in der im Markierungsraum eingenommenen Position. Die Transformationsmatrix bildet das 3D-Modell des Patienten, und insbesondere die Oberfläche des 3D-Modells, sowie die entsprechenden Bestrahlungsfelder aus dem virtuellen Koordinatensystem in das lokale Koordinatensystem ab. Dabei wird über die Referenzpunkte eine Patientenneupositionierung in dem Markierungsraum gegenüber seiner bei der Erstellung des virtuellen 3D-Modells seines Körpers eingenommenen Position berücksichtigt. Die Referenzpunkte dienen sozusagen als "Brücke" zwischen der bei der Erstellung des virtuellen 3D-Modells eingenommen Patientenposition und der Patientenposition bei der Markierung.

Erfindungsgemäß ist eine Markierung des Patienten in einem anderen Raum als dem für die Erstellung des virtuellen 3D-Modells genutzten Tomographiegeräts (z.B. CT-Gerät) möglich. Dadurch wird keine wertvolle Zeit in dem Tomographieraum für die Markierung beansprucht. Der so vorgeschlagene Markierungsraum ohne Tomographiegerät ist verhältnismäßig kostengünstig, da er nicht den strengen Strahlenschutzanforderungen genügen muss. Außerdem ist der Patient im Rahmen der erfindungsgemäßen Markierung keiner weiteren Strahlenbelastung ausgesetzt, da keine erneute Tomographieaufnahme erforderlich ist.

Selbstverständlich kann das Lasersystem auch zur Ermittlung der Koordinaten von mehr als zwei Referenzpunkten, beispielsweise drei Referenzpunkten, auf der Oberfläche des Patientenkörpers in dem lokalen Koordinatensystem ausgebildet sein. Die Anzahl der für die Bestimmung der Transformationsmatrix zu ermittelnden Referenzpunkte hängt von der Anzahl der Freiheitsgrade der Patientenbewegung zwischen seiner Position bei der Erstellung des virtuellen 3D-Modells und seiner Position bei der Markierung ab. Möglich ist grundsätzlich eine dreidimensionale Translation und dreidimensionale Rotation des Patienten. Zur Ermittlung der Matrix wird ein so genannter "Matching-Algorithmus" verwendet. Aus der Positionsveränderung des Patienten in den jeweiligen Freiheitsgraden wird die erforderliche Transformation aus der Patientenposition bei der Erstellung des virtuellen 3D-Modells in die Patientenposition bei der Markierung bestimmt. Daraus ergeben sich die Translationsparameter $t_X$, $t_Y$, $t_Z$ in den drei Raumrichtungen sowie die Rotationswinkel $\alpha$, $\beta$, $\gamma$ für die drei möglichen Rotationen um die entsprechenden X-, Y- und Z-Achsen der drei Raumrichtungen. Die Transformationsmatrix für die Korrektur der Bestrahlungsfeldkoordinaten sieht in ihrer allgemeinen Form wie folgt aus:

$$K = \begin{pmatrix} r_{11} & r_{12} & r_{13} & t_X \\ r_{21} & r_{22} & r_{23} & t_Y \\ r_{31} & r_{32} & r_{33} & t_Z \\ 0 & 0 & 0 & 1 \end{pmatrix}$$

Dabei stellen die Parameter $t_X$, $t_Y$ und $t_Z$ die dreidimensionale Translation des Patientenkörpers zwischen dem virtuellen und dem lokalen Koordinatensystem dar. Die weiteren Parameter $r_{11}$ bis $r_{33}$ stellen die dreidimensionale Rotation des Patientenkörpers dar. Diese Korrekturmatrix kann dann auf jeden Koordinatenpunkt der im Rahmen der virtuellen Bestrahlungsplanung errechneten Bestrahlungsfeldkoordinaten angewendet werden. Mathematisch liegt somit eine 3D-Transformation in einer homogenen Transformationsmatrix eines nicht deformierbaren Körpers vor, bestehend aus Rotation und Translation.

Die allgemeine Form der Transformation sieht wie folgt aus:

$$\begin{pmatrix} X' \\ Y' \\ Z' \\ 1 \end{pmatrix} = \begin{pmatrix} r_{11} & r_{12} & r_{13} & t_X \\ r_{21} & r_{22} & r_{23} & t_Y \\ r_{31} & r_{32} & r_{33} & t_Z \\ 0 & 0 & 0 & 1 \end{pmatrix} \cdot \begin{pmatrix} X \\ Y \\ Z \\ 1 \end{pmatrix}$$

oder

$$P' = R \cdot P + T$$

Dabei sind mit X', Y' und Z' bzw. P' die transformierten dreidimensionalen Koordinaten beispielsweise des Bestrahlungsfeldes gekennzeichnet. X, Y und Z bzw. P bezeichnen die Koordinaten des Bestrahlungsfeldes in dem virtuellen Koordinatensystem. R stellt die Rotation dar. Sind die Transformationsparameter nicht bekannt, ergibt sich aus dem Matrixprodukt für jeden dreidimensionalen Markierungspunkt auf der Patientenoberfläche ein Gleichungssystem mit insgesamt 6 linear voneinander unabhängigen Unbekannten, nämlich drei Rotationswinkeln $\alpha$, $\beta$, $\gamma$ sowie der Translationsparameter $t_X$, $t_Y$, $t_Z$. Für die Bestimmung der Transformationsmatrix ist in diesem Fall also die Bestimmung von drei dreidimensionalen Referenzpunkten auf der Oberfläche des Patientenkörpers in dem lokalen Koordinatensystem erforderlich.

Im Falle einer Rotation des Körpers nur um einen Winkel γ und einer Translation nur in einer horizontalen Ebene entlang der Achsen X und Y vereinfacht sich die Transformation in folgende Form:

$$\begin{pmatrix} X' \\ Y' \\ 1 \end{pmatrix} = \begin{pmatrix} r_{11} & r_{12} & t_X \\ r_{21} & r_{22} & t_Y \\ 0 & 0 & 1 \end{pmatrix} \cdot \begin{pmatrix} X \\ Y \\ 1 \end{pmatrix}$$

oder

$$P' = R \cdot P + T$$

Sind die Translationsparameter bekannt, müssen dann nur zwei Referenzpunkte für die Bestimmung der Matrix angesteuert werden. Da das Verfahren zur Bestimmung der Transformationsmatrix iterativ ist, kann eine Überbestimmung des Gleichungssystems aufgrund einer Ermittlung von mehr Referenzpunkten als erforderlich zu einer erhöhten Genauigkeit führen und aus diesem Grund vorteilhaft sein.

Die transformierten Koordinaten können anschließend dem Lasersystem zur Verfügung gestellt werden. Dann kann die Markierung der Bestrahlungsfelder und der Schnittpunkte mit der Haut des Zentralstrahls, der durch das Isozentrum geht, auf der Haut des Patienten erfolgen, beispielsweise manuell mittels eines Stifts. Die Form und Lage der so markierten Bestrahlungsfelder entspricht dann für die neue Patientenposition der im Rahmen der Bestrahlungsplanung berechneten Form und Lage der Felder. Die Auswerte- und Steuereinrichtung kann dazu ausgebildet sein, das Lasersystem so anzusteuern, dass die transformierten Koordinatenpunkte nacheinander mit mindestens einem Laserstrahl angefahren werden. Die Ansteuerung der Koordinatenpunkte kann beispielsweise über eine Fernbedienung erfolgen. Neben den Koordinaten des Umrisses des Bestrahlungsfeldes können insbesondere auch die Koordinaten der Schnittpunkte der Haut mit dem Zentralstrahl, der durch das Isozentrum geht, transformiert und der Markierungseinrichtung für die Markierung zur Verfügung gestellt werden.

Die Markierungseinrichtung weist mindestens einen Laser auf und kann mit der Auswerte- und Steuereinrichtung kombiniert ausgebildet sein. Die Auswerte- und Steuereinrichtung weist eine geeignete Software zur Durchführung ihrer Aufgaben auf. Damit die Referenzpunkte einfach angefahren werden können, können sie auf dem Patienten bei der Erstellung des 3D-Modells markiert werden und beispielsweise das Isozentrum einer zur Erstellung des virtuellen 3D-Modells genutzten Tomographieanlage (CT) kennzeichnen. Der Markierungsbereich des Patienten kann sich in dem Ursprung des lokalen Koordinatensystems der Vorrichtung befinden.

Die möglicherweise als Hautmarkierungspunkte im Rahmen einer Erstmarkierung in einem Tomographie-Raum auf die Patientenhaut aufgebrachten Referenzpunkte können die Schnittpunkte der Achsen des dem Patienten zugeordneten Patientenkoordinatensystems und des Tomographiesystems mit der Hautoberfläche des Patienten darstellen. Dabei kann bei dieser Erstmarkierung der Referenzpunkte auf dem Patienten der Ursprung des dem Patienten zugeordneten Patientenkoordinatensystems mit dem Ursprung des Tomographen-Koordinatensystems übereinstimmen. Wie oben erläutert, kann der Ursprung des Patientenkoordinatensystems in der Markierungsposition allerdings von dem Ursprung des Markierungssystems, also des lokalen Koordinatensystems der Vorrichtung abweichen. Diese Abweichung wird durch die Ermittlung der Referenzpunkte ermittelt und in der Transformationsmatrix berücksichtigt.

Das Lasersystem kann mindestens zwei jeweils eine Laserlinie erzeugende Laser umfassen. Die Koordinaten der Referenzpunkte sind im virtuellen Patientenkoordinatensystem bekannt. Die Ermittlung der lokalen Koordinaten der Referenzpunkte kann in besonders einfacher Weise durch eine Ermittlung der Translation der Laserlinien von dem Nullpunkt des lokalen Koordinatensystems zu den Referenzpunkten erfolgen. Der Nullpunkt des lokalen Koordinatensystems ist bekannt. Anhand der Translation sind dann auch die Koordinaten der angesteuerten Referenzpunkte bekannt. Durch das Lasersystem erfolgt eine entsprechende Positionsrückmeldung. Um die Referenzpunkte auch in dem beispielsweise im Rahmen einer CT-Aufnahme erstellten virtuellen 3D-Modell des Patientenkörpers sichtbar zu machen, können beispielsweise Kontrastmarkierungen (Schwermetallmarkierungen) an den Referenzpunkten auf den Patienten aufgebracht sein.

Nach der Tomographieaufnahme und der Erstmarkierung des Patienten an den Referenzpunkten wurde dem Patienten selbst auch ein Koordinatensystem zugeordnet. Dieses Patientenkoordinatensystems muss bei einer beliebigen Positionierung des Patienten nicht mit dem Isozentrum der Tomographie-Anlage übereinstimmen. So kann der Patient bezüglich der Anfangsposition sowohl einer Translation als auch einer Rotation unterzogen worden sein. Sofern jedoch die Koordinaten einer ausreichenden Anzahl von Referenzpunkten in dem lokalen Koordinatensystem und die virtuellen Koordinaten der Referenzpunkte in dem virtuellen Koordinatensystem bekannt sind, kann die Transformationsmatrix eindeutig bestimmt werden.

Gemäß einer weiteren Ausgestaltung kann das Lasersystem wenigstens zwei Laser-Projektionseinrichtungen umfassen, wobei die Referenzpunkte durch ein Schneiden von jeweils zwei von den Laser-Projektionseinrichtungen erzeugten Laserstrahlen auf der Patientenoberfläche ermittelt werden können. Es ist dann möglich, die Auswerte- und Steuereinrichtung dazu auszubilden, die Laser-Projektionseinrichtungen so anzusteuern, dass die transformierten Koordinaten bzw. Punkte, beispielsweise der Umriss des Bestrahlungsfelds, auf die dreidimensionale Oberfläche des Patientenkörpers projizierbar ist, indem mindestens ein von mindestens einer der Laser-Projektionseinrichtungen erzeugter Laserstrahl ausreichend schnell entlang der transformierten Koordinatenpunkte auf dem Patientenkörper geführt werden kann, so dass auf der Oberfläche der Eindruck einer geschlossenen Kontur (beispielsweise um das Bestrahlungsfeld) entsteht. Die Darstellung von Bestrahlungsfeldern mittels solcher Laser-Projektionseinrichtungen ist Gegenstand der parallelen deutschen Patentanmeldung der Anmelderin mit dem Aktenzeichen 10 2008 012 496.6. Die Position der Projektionseinrichtungen im Raum ist bekannt und definiert. Die Referenzpunkte können dazu beispielsweise mittels Reflektoren auf dem Patienten markiert sein. Werden nun zwei Projektorstrahlen auf einen Reflektorpunkt ausgerichtet, kann die Position (3D-Koordinaten) des Punktes im 3D-Raum anhand des Schnittpunkts der Strahlen bestimmt werden. Aus der Ermittlung der lokalen Koordinaten aller Referenzpunkte, die im virtuellen Koordinatensystem des virtuellen 3D-Modells des Patientenkörpers ebenfalls bekannt sind, können wiederum die Transformationsmatrixelemente bestimmt werden. Die Projektionseinrichtungen können jeweils mindestens zwei drehbare (galvanometrische) Spiegel aufweisen, mit denen die Laserstrahlen auf die Oberfläche des Patientenkörpers reflektiert und zu den Referenzpunkten bzw. entlang der Kontur des Bestrahlungsfelds geführt werden können. Anhand der Projektion der Kontur auf die Oberfläche kann dann wiederum z.B. per Hand mit einem Stift das Bestrahlungsfeld sowie die Schnittpunkte des Zentralstrahls, der durch das Isozentrum geht, mit der Haut auf der Haut markiert werden.

Es kann ein Oberflächenerfassungssystem vorgesehen sein, mit dem ein lokales 3D-Modell des Patientenkörpers in dem lokalen Koordinatensystem erstellt werden kann. Ein solches System kann beispielsweise ein Laser- oder ein Lichtstreifensystem sein. Gemäß einer weiteren Ausgestaltung der Erfindung kann das Lasersystem mindestens einen, eine Laserlinie erzeugenden Laser und mindestens eine Detektoreinrichtung aufweisen, wobei die Auswerte- und Steuereinrichtung dazu ausgebildet ist, mit dem Laser eine Laserlinie sukzessive über die Oberfläche des Patientenkörpers zu führen, das von dem Patientenkörper reflektierte Laserlicht mittels der Detektoreinrichtung zu detektieren und aus dem detektierten Laserlicht, beispielsweise nach dem Triangulationsverfahren, ein lokales 3D-Modell des Patientenkörpers in dem lokalen Koordinatensystem zu erstellen. Selbstverständlich kann dabei auch ein 3D-Modell eines interessierenden Teils des Patientenkörpers erstellt werden. Es können insbesondere zwei jeweils eine Laserlinie erzeugende Laser vorgesehen sein, die miteinander gekoppelt sind, um jede Region des Patientenkörpers erreichen zu können. Bei der Detektoreinrichtung kann es sich in besonders einfacher Weise um eine Kamera handeln. Auf Triangulation basierende Laser- oder Lichtschnittverfahren sind an sich bekannt. Dabei wird meist eine Kamera und eine feststehende Laserebene verwendet. Dort wo das Licht auf das zu vermessende Objekt auftritt, also in der sichtbaren Schnittebene zwischen Laserebene und Objektoberfläche, werden die Lichtstrahlen diffus in den Raum zurückreflektiert. Das reflektierte Licht wird von einer seitlich versetzten Kamera erfasst. Durch eine Kalibrierung der Kamera und einer weiteren Kalibrierung, in der die Anordnung aus Kamera und Laserebene zueinander bestimmt wird, lässt sich mit einer geeigneten Auswertesoftware eine Berechnung der Koordinaten der von dem Laserlicht beleuchteten Oberflächenpunkte vornehmen. Daraus wird eine zweidimensionale Information über die Form des Objekts erlangt. Ist eine dreidimensionale Vermessung des Objektes gewünscht, muss entweder die Laserlinie über die Objektoberfläche oder das Objekt in definierter Weise durch den Messbereich bewegt werden. Unabhängig von dem verwendeten Verfahren muss für jeden Zeitpunkt der Messung die Anordnung aus Laserebene und Kamera bekannt sein.

Die Laserebene ist motorisch entlang der Längsachse eines den Patienten beispielsweise aufnehmenden Lagerungstisches bewegbar. Der Laserscan kann erfolgen, indem eine beispielsweise quer zu dem Patienten ausgerichtete Laserlinie über den Patient bzw. die interessierende Patientenregion geführt wird. Durch gleichzeitige Erfassung der reflektierten Laserstrahlung mit der Detektoreinrichtung liegt ein Laserscanner vor. Bei dieser Ausgestaltung der Erfindung wird also eine Vielzahl, insbesondere eine unendliche Anzahl, von Referenzpunkten ermittelt, indem ein lokales 3D-Modell des Patientenkörpers erstellt wird. Referenzpunkte können dabei alle erfassten Oberflächenpunkte des Körpers sein. Diese Oberflächenpunkte sind wiederum als Referenzpunkte auch in dem virtuellen Koordinatensystem des virtuellen 3D-Modells bekannt. Die dabei erstellten 3D-Modelle des Patienten in dem lokalen Koordinatensystem können auch anderen Systemen, z.B. Diagnose oder Behandlungsgeräten, als Referenzoberfläche für eine Lagerungskontrolle des Patienten zur Verfügung gestellt werden.

Die Transformationsmatrix kann von der Auswerte- und Steuereinrichtung dann aus einem Vergleich des lokalen 3D-Modells des Patientenkörpers mit dem virtuellen 3D-Modell des Patientenkörpers ermittelt werden. Es wird also das mit dem Laserscan erstellte lokale 3D-Modell mit dem beispielsweise im Rahmen einer Tomographie-Aufnahme erstellten virtuellen 3D-Modell aus der Bestrahlungsplanung verglichen. Dabei kann der Abstand zwischen den beiden 3D-Modellen punktuell für eine Vielzahl von Punkten gemessen werden und die 3D-Modelle so in Überlappung gebracht werden, dass über die gesamte Oberfläche ein minimaler Abstand zwischen den Modellen erreicht wird (vorzugsweise Abstand Null). Aus der dazu erforderlichen Translation/Rotation des virtuellen 3D-Modells wird die Transformationsmatrix erstellt. Bei dieser Ausgestaltung der Erfindung, die sich durch eine besonders hohe Genauigkeit auszeichnet, ist eine Markierung der Referenzpunkte auf der Patientenhaut im Rahmen des Tomographie-Scans nicht zwingend erforderlich.

Das Lasersystem kann mindestens fünf jeweils eine Laserlinie erzeugende Laser umfassen, wobei zwei seitlich eines Lagerungstisches für den Patienten in der Höhe verstellbare Laser vorgesehen sind, die jeweils eine horizontale Linie entlang des Lagerungstische projizieren, und drei Laser oberhalb des Lagerungstisches vorgesehen sind, wobei ein Laser quer zur Längsrichtung des Lagerungstisches verschiebbar angeordnet ist und eine Laserlinie in Längsrichtung des Lagerungstisches projiziert, und wobei die beiden weiteren Laser in Längsrichtung des Lagerungstisches verschiebbar angeordnet sind und miteinander gekoppelt eine gemeinsame Laserlinie quer zur Längsrichtung des Lagerungstisches projizieren. Dieses an sich bekannte (DE 44 21 315 A1 oder DE 195 24 951 A1) und von der Anmelderin unter dem Namen "Dorado CT 4" vertriebene System kann erfindungsgemäß in neuer Weise eingesetzt werden. So können in besonders einfacher Weise die beiden über dem Tisch angeordneten und gekoppelten Laser für den Laserscanner eingesetzt werden. Durch die Kopplung der beiden Laser und die seitliche Anordnung längs der Patientenachse auf dem Behandlungstisch können auch noch solche Koordinaten auf der Haut dargestellt werden, die ansonsten unterhalb des Querdurchmessers des Patienten durch den Patienten selbst verschattet würden. Das System muss dann nur noch durch eine entsprechende Detektoreinrichtung, beispielsweise eine Kamera, ergänzt werden.

Die erfindungsgemäße Vorrichtung kann in einem anderen Raum angeordnet sein als eine zur Ermittlung des virtuellen 3D-Modells des Patientenkörpers genutzte Tomographieeinrichtung. Dadurch wird wertvolle Zeit in dem Tomographie-Raum gespart. Es können dann in beiden Räumen die gleichen Lagerungs- und Fixierungshilfen für den Patienten vorgesehen sein. Selbstverständlich kann die erfindungsgemäße Vorrichtung aber auch in demselben Raum angeordnet sein wie eine zur Erstellung des virtuellen 3D-Modells genutzte Tomographieeinrichtung (z.B. CT-Einrichtung).

Wenn die Referenzpunkte auf der Haut des Patientenkörpers im Rahmen des Tomographie-Scans und insbesondere der Erstellung des virtuellen 3D-Modells markiert werden, können zur Erstmarkierung im Tomographie-Raum feste Raumlaser verwendet werden, die so installiert sind, dass sie dem Tomographen-Isozentrum entsprechen. Eine Markierung der Referenzpunkte auf der Patientenhaut kann nach dem Anzeigen mit dem Laser mit einem Farbstift und gleichzeitig mit Kontrastteilen (Kugeln oder Kreuze oder ähnliches), die auf einer Tomographieaufnahme sichtbar sind, erfolgen.

Die erfindungsgemäße Vorrichtung kann zur Durchführung des erfindungsgemäßen Verfahrens geeignet sein.

Ein Ausführungsbeispiel der Erfindung wird nachfolgend anhand von Figuren näher erläutert. Es zeigen schematisch:

Fig. 1    eine erfindungsgemäße Vorrichtung gemäß einer ersten Ausgestaltung,

Fig. 2    eine erfindungsgemäße Vorrichtung gemäß einer zweiten Ausgestaltung, und

Fig. 3    eine erfindungsgemäße Vorrichtung gemäß einer dritten Ausgestaltung.

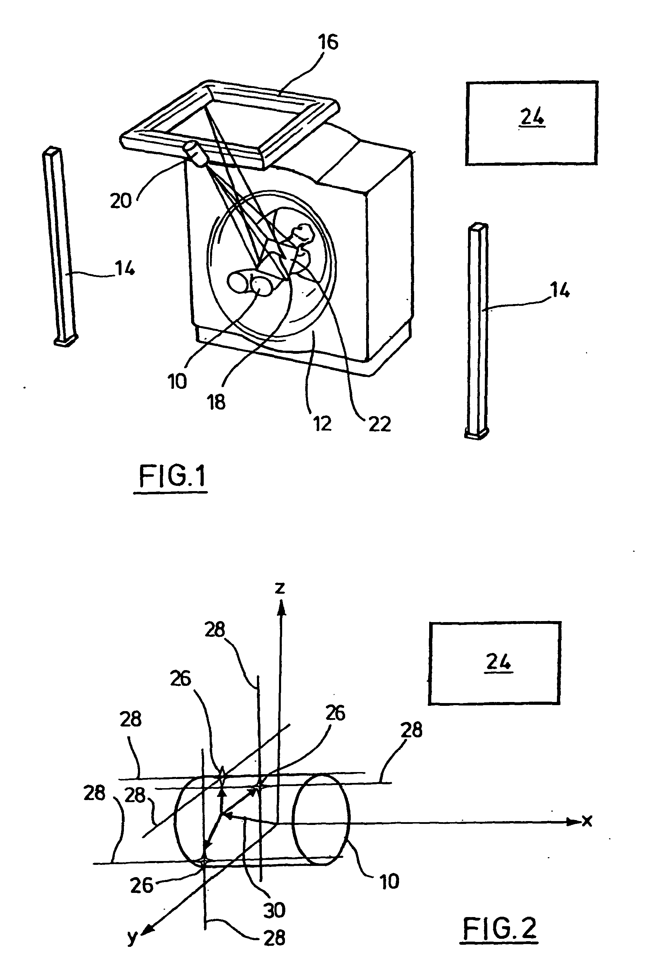

Soweit nichts anderes angegeben ist, bezeichnen in den Figuren gleiche Bezugszeichen gleiche Gegenstände. In Fig. 1 ist eine Vorrichtung zum Markieren eines Bestrahlungsfeldes auf der Oberfläche eines lediglich schematisch dargestellten Patientenkörpers 10 gezeigt. In dem Ausführungsbeispiel in Fig. 1 ist die Vorrichtung in demselben Raum angeordnet, wie ein Tomographie-Gerät 12 (z.B. CT-Gerät), mit dem ein virtuelles 3D-Modell des Patientenkörpers 10 für die Bestrahlungsplanung erstellt wird. Die Vorrichtung in Fig. 1 umfasst ein Lasersystem mit insgesamt fünf motorisch beweglichen Lasern. Dabei sind rechts und links seitlich eines nicht näher dargestellten Lagerungstisches für den Patienten zwei in der Höhe verstellbare Laser 14 vorgesehen, die jeweils eine horizontale Linie entlang des Tomographie-Tisches projizieren können. In einer über dem Tomographie-Tisch befindlichen Halteeinrichtung 16 sind drei weitere Laser vorgesehen (nicht näher dargestellt). Ein Laser ist dabei quer zur Tisch-Längsrichtung verschiebbar angeordnet und projiziert eine nicht dargestellte Linie in Längsrichtung des Tomographie-Tisches. Zwei weitere Laser in der Halterung 16 sind miteinander gekoppelt und projizieren eine Laserlinie 18 quer zur Längsachse des Tomographie-Tisches auf den Patientenkörper 10. Das Lasersystem umfasst weiterhin eine Detektoreinrichtung 20 in Form einer Kamera 20, vorliegend einer CCD-Kamera 20. Das von der Kamera 20 detektierte Sichtfeld ist in Fig. 1 mit 22 bezeichnet.

Mittels einer Auswerte- und Steuereinrichtung 24 wird die Laserlinie 18 sukzessive über den interessierenden Oberflächenbereich des Patientenkörpers 10 gerührt. Dabei bleibt die Laserlinie 18 in dem Detektionsbereich 22 der Kamera 20. Die von dem Patientenkörper 10 reflektierte Laserstrahlung wird von der Kamera 20 detektiert, Die Auswerte- und Steuereinrichtung 24 ermittelt aus dem Detektionsergebnis in an sich bekannter Weise nach dem Triangulationsverfahren ein 3D-Modell des Patientenkörpers in dem lokalen Koordinatensystem der Vorrichtung. Es wird also eine praktisch unendliche Anzahl von Referenzpunkten auf der Oberfläche des Patientenkörpers 10 in dem der Vorrichtung zugeordneten lokalen Koordinatensystem ermittelt. Anschließend wird von der Auswerte- und Steuereinrichtung 24 das so ermittelte lokale 3D-Modell des Patientenkörpers mit einem zuvor im Rahmen einer Bestrahlungsplanung anhand von mit dem Tomographie-Gerät erstellten Aufnahmen erstellten virtuellen 3D-Modell des Patientenkörpers 10 verglichen. Insbesondere wird dabei für eine Vielzahl von Oberflächenpunkten auf der Hülle der 3D-Modelle punktuell der Abstand zwischen den beiden 3D-Modellen gemessen und die 3D-Modelle mittels einer geeigneten Software so in Überlappung gebracht, dass über die gesamte Oberfläche der 3D-Modelle ein minimaler Abstand zwischen den 3D-Modellen besteht. Dabei wird das virtuelle 3D-Modell geeignet verschoben bzw. rotiert. Aus der dazu erforderlichen Translation bzw. Rotation des virtuellen 3D-Modells wird von der Auswerte- und Steuereinrichtung 24 eine Transformationsmatrix zur Transformation beliebiger Koordinaten aus dem virtuellen Koordinatensystem der Bestrahlungsplanung in das lokale Koordinatensystem ermittelt. Die Koordinaten des Bestrahlungsisozentrums und des Umrisses des im Rahmen der Bestrahlungsplanung ermittelten Bestrahlungsfeldes bzw. der ermittelten Bestrahlungsfelder werden anschließend mittels der Transformationsmatrix in das lokale Koordinatensystem transformiert. Die transformierten Koordinaten werden dann dem Lasersystem zur Verfügung gestellt. Beispielsweise mittels einer Fernbedienung können die transformierten Koordinaten durch die Laser des Lasersystems angefahren und von Hand mit einem Stift auf der Patientenhaut für die spätere Bestrahlung markiert werden.

Fig. 2 zeigt eine alternative Ausgestaltung der erfindungsgemäßen Vorrichtung. In diesem Fall weist das Lasersystem mindestens zwei nicht näher dargestellte, jeweils eine Laserlinie erzeugende Laser auf. Auf dem Patientenkörper 10 sind im Rahmen der Bestrahlungsplanung drei Referenzpunkte 26 markiert worden. Diese sind vorliegend sowohl mittels eines Stifts visuell, als auch mittels entsprechender Schwermetallmarkierungen für eine CT-Aufnahme einer Bestrahlungsplanung sichtbar gemacht worden. Die Referenzpunkte 26 können von dem Lasersystem durch die Laserlinien 28 angefahren werden. Die Koordinaten der Referenzpunkte in dem lokalen Koordinatensystem der Vorrichtung werden dabei durch eine Ermittlung der Translation der Laserlinien aus dem Nullpunkt des lokalen Koordinatensystems zu den Referenzpunkten 26 ermittelt. Somit sind die Koordinaten der Referenzpunkte 26 in dem lokalen Koordinatensystem bekannt.

Außerdem sind die virtuellen Koordinaten der Referenzpunkte 26 in dem virtuellen Koordinatensystem des im Rahmen der Bestrahlungsplanung erstellten 3D-Modells des Patientenkörpers aufgrund der Erkennbarkeit auf der CT-Aufnahme ebenfalls bekannt. Mittels der Auswerte- und Steuereinrichtung 24 kann auf dieser Grundlage die Transformationsmatrix zur Transformation beliebiger Koordinaten aus dem virtuellen Koordinatensystem X, Y, Z des virtuellen 3D-Modells des Patientenkörpers in das der Vorrichtung zugeordnete lokale Koordinatensystem ermittelt werden. Für die Transformation wird ein entsprechender die Translation zwischen den virtuellen und lokalen Koordinatensystemen darstellender Verschiebungsvektor 30 verwendet. Die eine dreidimensionale Rotation des Patienten darstellenden Rotationsparameter werden anhand der Koordinaten der Referenzpunkte ermittelt.

Die Koordinaten des Isozentrums und des Umrisses des berechneten Soll-Bestrahlungsfelds können dann mit der Transformationsmatrix in das der Vorrichtung zugeordnete lokale Koordinatensystem transformiert und anschließend dem Lasersystem zur Verfügung gestellt werden. Die transformierten Koordinaten können dann wiederum beispielsweise durch ein Schneiden von zwei Laserlinien 28 sukzessive auf der Haut des Patienten angefahren und manuell beispielsweise mit einem Stift markiert werden.

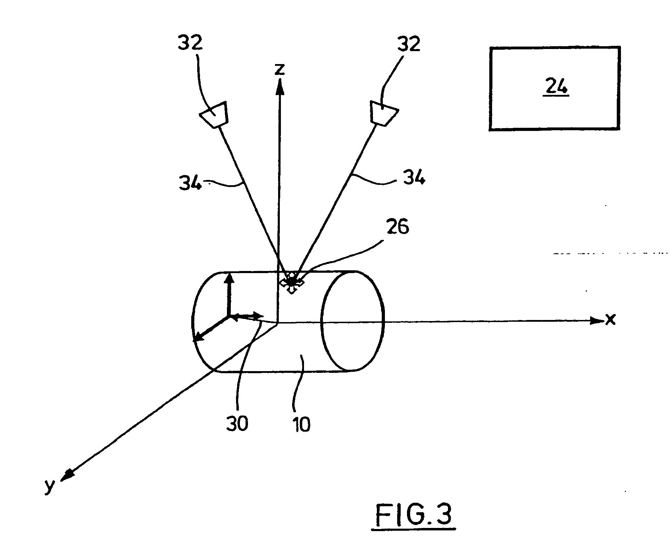

In Fig. 3 ist eine weitere Ausgestaltung der erfindungsgemäßen Vorrichtung dargestellt. Das in Fig. 3 dargestellte Lasersystem weist zwei Laserprojektionseinrichtungen 32 auf. Durch ein Schneiden von jeweils zwei von den Laserprojektionseinrichtungen 32 erzeugten Laserstrahlen 34 auf der Oberfläche des Patientenkörpers 10 können auf der Patientenkörperoberfläche markierte Referenzpunkte 26 angefahren werden. Aufgrund der definierten Positionen der Laser-Projektionseinrichtungen 32 in dem Raum der Vorrichtung können auf diese Weise die Koordinaten der Referenzpunkte 26 im dreidimensionalen Raum ermittelt werden. Dies übernimmt die Auswerte- und Steuereinrichtung 24. Da wiederum die Koordinaten der Referenzpunkte 26 ebenfalls in dem virtuellen 3D-Modell des Patientenkörpers aus der CT-Bestrahlungsplanung bekannt sind, kann die Auswerte- und Steuereinrichtung 24 auf dieser Grundlage die Transformationsmatrix zur Transformation beliebiger Koordinaten aus dem virtuellen Koordinatensystem in das lokale Koordinatensystem ermitteln. Anschließend können von der Auswerte- und Steuereinrichtung 24 mit der Transformationsmatrix die Koordinaten des Isozentrums und des Umrisses des Bestrahlungsfeldes bzw. der Bestrahlungsfelder in das lokale Koordinatensystem transformiert und dem Lasersystem zur Verfügung gestellt werden.

Bei dem in Fig. 3 dargestellten Ausführungsbeispiel werden die Laser-Projektionseinrichtungen 32 von der Auswerte- und Steuereinrichtung 24 für die Markierung so angesteuert, dass das Bestrahlungsfeld auf die dreidimensionale Oberfläche des Patientenkörpers 10 projiziert wird. Dabei wird insbesondere mindestens eine der Projektionseinrichtungen 32 so angesteuert, dass der von dieser Einrichtung 32 erzeugte Laserstrahl 34 ausreichend schnell entlang der transformierten Koordinaten auf der Oberfläche des Patientenkörpers 10 geführt wird, so dass auf der Oberfläche 10 der Eindruck einer geschlossenen Kontur um das Bestrahlungsfeld entsteht. Das so auf die Oberfläche des Patientenkörpers 10 projizierte Strahlungsfeld kann dann beispielsweise manuell markiert werden. Befindet sich das Lasersystem aus Fig. 3 bereits in dem für die Bestrahlung eingesetzten Bestrahlungsraum, kann das projizierte Bestrahlungsfeld sogar direkt mit dem den Strahl des Bestrahlungsgeräts visualisierenden Lichtfeld verglichen werden. Eine Markierung des Bestrahlungsfelds auf der Patientenkörperoberfläche mittels eines Stifts oder ähnlichem ist dann nicht mehr erforderlich.

Mit sämtlichen erfindungsgemäßen Vorrichtungen ist in einfacher, kostengünstiger und medizinisch unbedenklicher Weise eine Markierung eines ein Soll-Schnittfeld eines Behandlungsstrahls mit der Patientenkörperoberfläche darstellenden Bestrahlungsfelds für eine Strahlungstherapie, beispielsweise zur Krebsbehandlung, möglich. Dabei werden jeweils die im Rahmen der Bestrahlungsplanung anhand eines mit einem Tomographiegerät erzeugten virtuellen 3D-Modells des Patienten berechneten Koordinaten des Bestrahlungsfelds aus dem virtuellen Koordinatensystem in das lokale Koordinatensystem transformiert. Anhand der Ermittlung der Koordinaten der Referenzpunkte auf der Oberfläche des Patientenkörpers wird die Positionsänderung des Patienten gegenüber seiner Lage bei der Tomographieaufnahme berücksichtigt. Es ist somit nicht erforderlich, den Patienten in dem Markierungsraum neu zu positionieren. Vielmehr werden die Koordinaten des Bestrahlungsfelds entsprechend seiner Positionsänderung transformiert.

**Patentansprüche**

1. Vorrichtung zur Markierung eines anhand eines virtuellen 3D-Modells eines Patientenkörpers erstellten Bestrahlungsfeldes auf der Oberfläche des Patientenkörpers,
   mit einem Lasersystem zur Ermittlung der Koordinaten mindestens zweier Referenzpunkte (26) auf der Oberfläche des Patientenkörpers (10) in einem der Vorrichtung zugeordneten lokalen Koordinatensystem,
   mit einer Auswerte- und Steuereinrichtung (24), die dazu ausgebildet ist, aus den Koordinaten der Referenzpunkte (26) in dem lokalen Koordinatensystem und aus Koordinaten der Referenzpunkte (26) in einem virtuellen Koordinatensystem des virtuellen 3D-Modells des Patientenkörpers (10) eine Transformationsmatrix zur Transformation beliebiger Koordinaten aus dem virtuellen Koordinatensystem in das lokale Koordinatensystem zu ermitteln,
   und **gekennzeichnet dadurch, dass** die Auswerte- und Steuereinrichtung (24) weiter dazu ausgebildet ist, mit der Transformationsmatrix Koordinaten aus dem virtuellen Koordinatensystem in das lokale Koordinatensystem zu transformieren und die transformierten Koordinaten dem Lasersystem zur Verfügung zu stellen, wobei die Auswerte- und Steuereinrichtung (24) dazu ausgebildet ist, das Lasersystem so anzusteuern, dass die transformierten Koordinatenpunkte nacheinander mit mindestens einem Laserstrahl angefahren werden.

2. Vorrichtung nach Anspruch 1, **dadurch gekennzeichnet, dass** die Auswerte- und Steuereinrichtung (24) dazu ausgebildet ist, die Koordinaten zumindest des Bestrahlungsisozentrums aus dem virtuellen Koordinatensystem in das lokale Koordinatensystem zu transformieren und die transformierten Koordinaten dem Lasersystem zur Verfügung zu stellen.

3. Vorrichtung nach Anspruch 1 oder nach Anspruch 2, **dadurch gekennzeichnet, dass** die Auswerte- und Steuereinrichtung (24) dazu ausgebildet ist, mit der Transformationsmatrix die Koordinaten zumindest des Umrisses des Bestrahlungsfeldes aus dem virtuellen Koordinatensystem in das lokale Koordinatensystem zu transformieren und die transformierten Koordinaten dem Lasersystem zur Verfügung zu stellen.

4. Vorrichtung nach einem der vorhergehenden Ansprüche, **dadurch gekennzeichnet, dass** das Lasersystem mindestens zwei jeweils eine Laserlinie (28) erzeugende Laser umfasst.

5. Vorrichtung nach Anspruch 4, **dadurch gekennzeichnet, dass** die lokalen Koordinaten der Referenzpunkte (26) von dem Lasersystem durch eine Ermittlung der Translation der Laserlinien (28) von dem Nullpunkt des lokalen Koordinatensystems zu den Referenzpunkten (26) ermittelt werden können.

6. Vorrichtung nach einem der Ansprüche 1 bis 3, **dadurch gekennzeichnet, dass** das Lasersystem wenigstens zwei Laserprojektionseinrichtungen (32) umfasst, wobei die Referenzpunkte (26) durch ein Schneiden von jeweils zwei von den Laserprojektionseinrichtungen (32) erzeugten Laserstrahlen (24) auf der Patientenoberfläche (10) ermittelt werden können.

7. Vorrichtung nach Anspruch 6, **dadurch gekennzeichnet, dass** die Auswerte- und Steuereinrichtung (24) dazu ausgebildet ist, die Laserprojektionseinrichtungen (32) so anzusteuern, dass die transformierten Koordinaten auf die dreidimensionale Oberfläche des Patientenkörpers (10) projizierbar sind, indem mindestens ein von mindestens einer der Laserprojektionseinrichtungen (32) erzeugter Laserstrahl (34) ausreichend schnell entlang der transformierten Koordinatenpunkte auf der Oberfläche des Patientenkörpers (10) geführt werden kann, so dass auf der Oberfläche der Eindruck einer geschlossenen Kontur entsteht.

8. Vorrichtung nach einem der vorhergehenden Ansprüche, **dadurch gekennzeichnet, dass** die Referenzpunkte (26) auf der Haut des Patientenkörpers (10) markiert sind.

9. Vorrichtung nach einem der Ansprüche 1 bis 7, **dadurch gekennzeichnet, dass** ein Oberflächenerfassungssystem vorgesehen ist, mit dem ein lokales 3D-Modell des Patientenkörpers (10) in dem lokalen Koordinatensystem erstellt werden kann.

10. Vorrichtung nach einem der Ansprüche 1 bis 7 oder nach Anspruch 9, **dadurch gekennzeichnet, dass** das Lasersystem mindestens einen, eine Laserlinie erzeugenden Laser und mindestens eine Detektoreinrichtung (20) aufweist, wobei die Auswerte- und Steuereinrichtung (24) dazu ausgebildet ist, mit dem Laser eine Laserlinie (18) sukzessive über die Oberfläche des Patientenkörpers (10) zu führen, das von dem Patientenkörper (10) reflektierte Laserlicht mit der Detektoreinrichtung (20) zu detektieren und aus dem detektierten Laserlicht ein lokales 3D-Modell des Patientenkörpers (10) in dem lokalen Koordinatensystem zu erstellen.

11. Vorrichtung nach Anspruch 10, **dadurch gekennzeichnet, dass** die Auswerte- und Steuereinrichtung (24) dazu ausgebildet ist, die Transformationsmatrix aus einem Vergleich des lokalen 3D-Modells des Patientenkörpers (10) mit dem virtuellen 3D-Modell des Patientenkörpers (10) zu ermitteln.

12. Vorrichtung nach einem der vorhergehenden Ansprüche, **dadurch gekennzeichnet, dass** das Lasersystem mindestens fünf jeweils eine Laserlinie erzeugende Laser (14) umfasst, wobei zwei seitlich eines Lagerungstisches für den Patienten in der Höhe verstellbare Laser (14) vorgesehen sind, die jeweils eine horizontale Linie entlang des Lagerungstisches projizieren, und drei Laser oberhalb des Lagerungstisches vorgesehen sind, wobei ein Laser quer zur Längsrichtung des Lagerungstisches verschiebbar angeordnet ist und eine Laserlinie in Längsrichtung des Lagerungstisches projiziert, und wobei die beiden weiteren Laser in Längsrichtung des Lagerungstisches verschiebbar angeordnet sind und miteinander gekoppelt eine gemeinsame Laserlinie (18) quer zur Längsrichtung des Lagerungstisches projizieren.

13. Vorrichtung nach einem der vorhergehenden Ansprüche, **dadurch gekennzeichnet, dass** die Vorrichtung in einem anderen Raum angeordnet ist als eine zur Ermittlung des virtuellen 3D-Modells des Patientenkörpers (10) genutzte Tomographieeinrichtung (12).

14. Verfahren zur Markierung eines anhand eines virtuellen 3D-Modells eines Patientenkörpers erstellten Bestrahlungsfeldes auf der Oberfläche des Patientenkörpers, bei dem die Koordinaten mindestens zweier Referenzpunkte (26) auf der Oberfläche des Patientenkörpers (10) in einem der Vorrichtung zugeordneten lokalen Koordinatensystem ermittelt werden, bei dem aus den Koordinaten der Referenzpunkte (26) in dem lokalen Koordinatensystem und aus Koordinaten der Referenzpunkte (26) in einem virtuellen Koordinatensystem des virtuellen 3D-Modells des Patientenkörpers (10) eine Transformationsmatrix zur Transformation beliebiger Koordinaten aus dem virtuellen Koordinatensystem in das lokale Koordinatensystem ermittelt wird, und **gekennzeichnet dadurch, dass** mit der Transformationsmatrix Koordinaten aus dem virtuellen Koordinatensystem in das lokale Koordinatensystem transformiert werden und für die Markierung zur Verfügung gestellt werden, und wobei die transformierten Koordinatenpunkte nacheinander mit mindestens einem Laserstrahl angefahren werden.

15. Verfahren nach Anspruch 14, **dadurch gekennzeichnet, dass** mit der Transformationsmatrix die Koordinaten zumindest des Bestrahlungsisozentrums aus dem virtuellen Koordinatensystem in das lokale Koordinatensystem transformiert werden und für die Markierung zur Verfügung gestellt werden.

16. Verfahren nach Anspruch 14 oder nach Anspruch 15, **dadurch gekennzeichnet, dass** mit der Transformationsmatrix die Koordinaten zumindest des Umrisses des Bestrahlungsfelds aus dem virtuellen Koordinatensystem in das lokale Koordinatensystem transformiert werden und für die Markierung zur Verfügung gestellt werden.

17. Verfahren nach einem der Ansprüche 14 bis 16, **dadurch gekennzeichnet, dass** das Lasersystem mindestens zwei jeweils eine Laserlinie (28) erzeugende Laser umfasst.

18. Verfahren nach Anspruch 17, **dadurch gekennzeichnet, dass** die lokalen Koordinaten der Referenzpunkte (26) durch eine Ermittlung der Translation der Laserlinien (28) von dem Nullpunkt des lokalen Koordinatensystems zu den Referenzpunkten (26) ermittelt werden.

19. Verfahren nach einem der Ansprüche 14 bis 16, **dadurch gekennzeichnet, dass** die Referenzpunkte (26) durch ein Schneiden von jeweils zwei von mindestens zwei Laserprojektionseinrichtungen (32) erzeugten Laserstrahlen (34) auf der Patientenoberfläche ermittelt werden können.

20. Verfahren nach Anspruch 19, **dadurch gekennzeichnet, dass** die transformierten Koordinaten auf die dreidimensionale Oberfläche des Patientenkörpers (10) projiziert werden, indem mindestens ein von mindestens einer der Laserprojektionseinrichtungen (32) erzeugter Laserstrahl (34) ausreichend schnell entlang der transformierten Koordinatenpunkte auf der Oberfläche des Patientenkörpers (10) geführt wird, so dass auf der Oberfläche der Eindruck einer geschlossenen Kontur entsteht.

21. Verfahren nach einem der Ansprüche 14 bis 20, **dadurch gekennzeichnet, dass** die Referenzpunkte (26) auf der Haut des Patientenkörpers (10) bei der Erstellung des virtuellen 3D-Modells des Patientenkörpers (10) markiert werden.

22. Verfahren nach einem der Ansprüche 14 bis 20, **dadurch gekennzeichnet, dass** mit einem Oberflächenerfassungssystem ein lokales 3D-Modell des Patientenkörpers (10) in dem lokalen Koordinatensystem erstellt wird.

23. Verfahren nach einem der Ansprüche 14 bis 20 oder nach Anspruch 22, **dadurch gekennzeichnet, dass** mit mindestens einem Laser eine Laserlinie (18) sukzessive über die Oberfläche des Patientenkörpers (10) geführt wird, das von dem Patientenkörper (10) reflektierte Laserlicht mit mindestens einer Detektoreinrichtung (20) detektiert wird und aus dem detektierten Laserlicht ein lokales 3D-Modell des Patientenkörpers (10) in dem lokalen Koordinatensystem erstellt wird.

24. Verfahren nach Anspruch 23, **dadurch gekennzeichnet, dass** die Transformationsmatrix aus einem Vergleich des lokalen 3D-Modells des Patientenkörpers (10) mit dem virtuellen 3D-Modell des Patientenkörpers (10) ermittelt wird.

25. Verfahren nach einem der Ansprüche 14 bis 24, **dadurch gekennzeichnet, dass** mindestens fünf jeweils eine Laserlinie erzeugende Laser (14) vorgesehen sind, wobei zwei seitlich eines Lagerungstisches für den Patienten in der Höhe verstellbar angeordnete Laser (14) vorgesehen sind, die jeweils eine horizontale Linie entlang des Lagerungstisches projizieren, und die übrigen Laser oberhalb des Lagerungstisches angeordnet sind, wobei ein Laser quer zur Längsrichtung des Lagerungstisches verschiebbar angeordnet ist und eine Laserlinie in Längsrichtung des Lagerungstisches projiziert, und wobei die beiden weiteren Laser in Längsrichtung des Lagerungstisches verschiebbar angeordnet sind und miteinander gekoppelt eine gemeinsame Laserlinie (18) quer zur Längsrichtung des Lagerungstisches projizieren.

26. Verfahren nach einem der Ansprüche 14 bis 25, **dadurch gekennzeichnet, dass** das Verfahren in einem anderen Raum durchgeführt wird als die Ermittlung des virtuellen 3D-Modells des Patientenkörpers (10).

**Claims**

1. An apparatus for marking an irradiation field on the surface of a patient's body, which was produced by means of a virtual 3D model of the patient's body, with a laser system for determining the coordinates of at least two reference points (26) on the surface of the patient's body (10) in a local coordinate system assigned to the apparatus, with an analysing and control unit (24) which is realised to determine a transformation matrix for the transformation of arbitrary coordinates from the virtual coordinate system into the local coordinate system from the coordinates of the reference points (26) in the local coordinate system and from coordinates of the reference points (26) in a virtual coordinate system of the virtual 3D model of the patient's body (10), and **characterized in that** the analysing and control unit (24) is further realised to transform coordinates from the virtual coordinate system into the local coordinate system with the transformation matrix, and to provide the transformed coordinates to the laser system wherein the analysing and control unit (24) is realised to drive the laser system such that the transformed coordinate points are approached one after the other with at least one laser beam.

2. An apparatus according to claim 1, **characterised in that** the analysing and control unit (24) is realised to transform the coordinates of at least the irradiation isocentre from the virtual coordinate system into the local coordinate system, and to provide the transformed coordinates to the laser system.

3. An apparatus according to claim 1 or according to claim 2, **characterised in that** the analysing and control unit (24) is realised to transform the coordinates of at least the outline of the irradiation field from the virtual coordinate system into the local coordinate system with the transformation matrix, and to provide the transformed coordinates to the laser system.

4. An apparatus according to any one of the preceding claims, **characterised in that** the laser system comprises at least two lasers, each one thereof generating one laser line (28) at a time.

5. An apparatus according to claim 4, **characterised in that** the local coordinates of the reference points (26) can be determined by the laser system through a determination of the translation of the laser lines (28) from the zero point of the local coordinate system to the reference points (26).

6. An apparatus according to any one of claims 1 to 3, **characterised in that** the laser system comprises at least two laser projection devices (32), wherein the reference points (26) can be determined by an intersection of two laser beams (24) at a time, generated by the laser projection devices (32), on the surface of the patient's body (10).

7. An apparatus according to claim 6, **characterised in that** the analysing and control unit (24) is realised to drive the laser projection devices (32) such that the transformed coordinates can be projected to the three-dimensional surface of the patient's body (10), while at least one laser beam (34) generated by at least one of the laser projection devices (32) can be guided along the transformed coordinate points on the surface of the patient's body (10) sufficiently rapidly, so that the impression of a closed contour results on the surface.

8. An apparatus according to any one of the preceding claims, **characterised in that** the reference points (26) are marked on the skin of the patient's body (10).

9. An apparatus according to any one of claims 1 to 7, **characterised in that** a surface mapping system is provided, by which a local 3D-model of the patient's body (10) can be produced in the local coordinate system.

10. An apparatus according to any one of claims 1 to 7 or according to claim 9, **characterised in that** the laser system has at least one laser generating a laser line and at least one detector device (20), wherein the analysing and control unit (24) is realised to guide a laser line (18) successively across the surface of the patient's body (10), to detect the laser light reflected by the patient's body (10) with the detector device (20) and to produce a local 3D-model of the patient's body (10) in the local coordinate system from the detected laser light.

11. An apparatus according to claim 10, **characterised in that** the analysing and control unit (24) is realised to determine the transformation matrix by a comparison of the local 3D-model of the patient's body (10) with the virtual 3D-model of the patient's body (10).

12. An apparatus according to any one of the preceding claims, **characterised in that** the laser system comprises at least five lasers (14), each one thereof generating one laser line at a time, wherein two lasers (14) adjustable in the height are provided on the sides of a positioning table for the patient, which each one at a time project one horizontal line along the positioning table, and three lasers are provided above the positioning table, wherein one laser is arranged to be movable transversely to the longitudinal direction of the positioning table and projects a laser line in the longitudinal direction of the positioning table, and wherein the two other lasers are arranged to be movable in the longitudinal direction of the positioning table and project a common laser line (18) transversely to the longitudinal direction of the positioning table, being coupled with each other.

13. An apparatus according to any one of the preceding claims, **characterised in that** the apparatus is disposed in another room than a tomography apparatus (12) used for determining the virtual 3D-model of the patient's body (10).

14. A method for marking an irradiation field on the surface of the patient's body, which was produced by means of a virtual 3D model of a patient's body, wherein the coordinates of at least two reference points (26) on the surface of the patient's body (10) are determined in a local coordinate system assigned to the apparatus,
wherein a transformation matrix for the transformation of arbitrary coordinates from the virtual coordinate system into the local coordinate system is determined from the coordinates of the reference points (26) in the local coordinate system and
from coordinates of the reference points (26) in a virtual coordinate system of the virtual 3D model of the patient's body (10),
and **characterized in that** coordinates are transformed from the virtual coordinate system into the local coordinate system with the transformation matrix, and are provided for the marking and wherein the transformed coordinate points are approached one after the other with at least one laser beam.

15. A method according to claim 14, **characterised in that** the coordinates of at least the irradiation isocentre are transformed from the virtual coordinate system into the local coordinate system with the transformation matrix, and are provided for the marking.

16. A method according to claim 14 or according to claim 15, **characterised in that** the coordinates of at least the outline of the irradiation field are transformed from the virtual coordinate system into the local coordinate system with the transformation matrix, and are provided for the marking.

17. A method according to any one of claims 14 to 16, **characterised in that** the laser system comprises at least two lasers, each one thereof generating one laser line (28) at a time.

18. A method according to claim 17, **characterised in that** the local coordinates of the reference points (26) are determined by a determination of the translation of the laser lines (28) from the zero point of the local coordinate system to the reference points (26)

19. A method according to any one of claims 14 to 16, **characterised in that** the reference points (26) are determined by an intersection of two laser beams (34) at a time, generated by at least two laser projection devices (32), on the patient's surface.

20. A method according to claim 19, **characterised in that** the transformed coordinates are projected to the three-dimensional surface of the patient's body (10) while at least one laser beam (34) generated by at least one of the laser projection devices (32) is guided along the transformed coordinate points on the surface of the patent's body (10) sufficiently rapidly, so that the impression of a closed contour results on the surface.

21. A method according to any one of claims 14 to 20, **characterised in that** the reference points (26) are marked on the skin of the patient's body (10) in the production of the virtual 3D-model of the patient's body (10).

22. A method according to any one of claims 14 to 20, **characterised in that** a local 3D-model of the patient's body (10) is produced in the local coordinate system by a surface mapping system.

23. A method according to any one of claims 14 to 20 or according to claim 22, **characterised in that** a laser line (18) is guided successively across the surface of the patient's body (10) with at least one laser, the laser light reflected by the patient's body (10) is detected by at least one detector device (20) and a local 3D-model of the patient's body (10) in the local coordinate system is produced from the detected laser light.

24. A method according to claim 23, **characterised in that** the transformation matrix is determined by a comparison of the local 3D-model of the patient's body (10) with the virtual 3D-model of the patient's body (10).

25. A method according to any one of claims 14 to 24, **characterised in that** at least five lasers (14) are provided, each one generating one laser line at a time, wherein two lasers (14) adjustable in the height are provided on the sides of a positioning table for the patient, which each one at a time project one horizontal line along the positioning table, and the remaining lasers are provided above the positioning table, wherein one laser is arranged to be movable transversely to the longitudinal direction of the positioning table and projects a laser line in the longitudinal direction of the positioning table, and wherein the two other lasers are arranged to be movable in the longitudinal direction of the positioning table and project a common laser line (18) transversely to the longitudinal direction of the positioning table, being coupled with each other.

26. A method according to any one of claims 14 to 25, **characterised in that** the method is performed in another room than the determination of the virtual 3D-model of the patient's body (10).

**Revendications**

1. Dispositif de marquage d'un champ de rayonnement produit au moyen d'un modèle tridimensionnel virtuel du corps d'un patient sur la surface du corps du patient,
avec un système laser destiné à déterminer les coordonnées d'au moins deux points de référence (26) sur la surface du corps du patient (10) dans un système de coordonnées local associé au dispositif,
avec un dispositif d'évaluation et de commande (24) conçu pour, à partir des coordonnées des points de référence (26) dans le système de coordonnées local et a partir de coordonnées des points de référence (26) dans un système de coordonnées virtuel du modèle tridimensionnel virtuel du corps du patient (10), déterminer une matrice de transformation pour la transformation de coordonnées quelconques du système de coordonnées virtuel vers le système de coordonnées local,
et **caractérisé en ce que** le dispositif d'évaluation et de commande (24) est en outre conçu pour, à l'aide de la matrice de transformation, transformer des coordonnées du système de coordonnées virtuel vers le système de coordonnées local, et mettre les coordonnées transformées à disposition du système laser, dans lequel le dispositif d'évaluation et de commande (24) est conçu pour commander le système laser de telle sorte que les points de coordonnées transformés sont parcourus en séquence à l'aide d'au moins un rayon laser.

2. Dispositif selon la revendication 1, **caractérisé en ce que** le dispositif d'évaluation et de commande (24) est en outre conçu pour transformer les coordonnées au moins de l'isocentre de rayonnement du système de coordonnées virtuel vers le système de coordonnées local et pour mettre les coordonnées transformées à disposition du système laser.

3. Dispositif selon la revendication 1 ou selon la revendication 2, **caractérisé en ce que** le dispositif d'évaluation et de commande (24) est en outre conçu pour, à l'aide de la matrice de transformation, transformer les coordonnées au moins du contour du champ de rayonnement du système de coordonnées virtuel vers le système de coordonnées local et pour mettre les coordonnées transformées à disposition du système laser.

**4.** Dispositif selon l'une quelconque des revendications qui précèdent, **caractérisé en ce que** le système laser comporte au moins deux lasers qui génèrent chacun une ligne laser (28).

**5.** Dispositif selon la revendication 4, **caractérisé en ce que** les coordonnées locales des points de référence (26) peuvent être déterminées par le système laser à l'aide d'une détermination de la translation des lignes laser (28) depuis le point zéro du système de coordonnées local vers les points de référence (26).

**6.** Dispositif selon l'une quelconque des revendications 1 à 3, **caractérisé en ce que** le système laser comporte au moins deux dispositifs de projection laser (32), dans lequel les points de référence- (26) peuvent être déterminés par une intersection de deux rayons laser (24) générés par les deux dispositifs de projection laser (32) sur la surface du patient (10).

**7.** Dispositif selon la revendication 6, **caractérisé en ce que** le dispositif d'évaluation et de commande (24) est en outre conçu pour commander les dispositifs de projection laser (32) de telle sorte que les coordonnées transformées peuvent être projetées sur la surface tridimensionnelle du corps du patient (10), moyennant quoi au moins un rayon laser (34) généré par au moins un des dispositifs de projection laser (32) peut être déplacé suffisamment vite le long des points de coordonnées transformés sur la surface du corps du patient (10) pour que l'impression d'un contour fermé soit obtenue sur la surface.

**8.** Dispositif selon l'une quelconque des revendications qui précèdent, **caractérisé en ce que** les points de référence (26) sont marqués sur la peau du corps du patient (10).

**9.** Dispositif selon l'une quelconque des revendications 1 à 7, **caractérisé en ce qu'**un système de détection de surface est pourvu, grâce auquel un modèle tridimensionnel local du corps du patient (10) peut être produit dans le système de coordonnées local.

**10.** Dispositif selon l'une des revendications 1 à 7 ou selon la revendication 9, **caractérisé en ce que** le système laser comporte au moins un laser générant une ligne laser et au moins un dispositif de détection (20), dans lequel le dispositif d'évaluation et de commande (24) est conçu pour tracer successivement une ligne laser (18) avec le laser sur la surface du corps du patient (10), pour détecter la lumière laser réfléchie par le corps du patient (10) avec le dispositif de détection (20), et pour produire un modèle tridimensionnel local du corps du patient (10) dans le système de coordonnées local à partir de la lumière laser détectée.

**11.** Dispositif selon la revendication 10, **caractérisé en ce que** le dispositif d'évaluation et de commande (24) est en outre conçu pour fournir la matrice de transformation à partir d'une comparaison du modèle tridimensionnel local du corps du patient (10) avec le modèle tridimensionnel virtuel du corps du patient (10).

**12.** Dispositif selon l'une quelconque des revendications qui précèdent, **caractérisé en ce que** le système laser comporte au moins cinq lasers (14) qui génèrent chacun une ligne laser, dans lequel deux lasers (14) réglables en hauteur sur le côté d'une table de support pour le patient sont pourvus, qui projettent chacun une ligne horizontale le long de la table de support, et trois lasers sont pourvus au-dessus de la table de support, dans lequel un laser est agencé de manière coulissante transversalement par rapport à la direction longitudinale de la table de support et projette une ligne laser en direction longitudinale de la table de support, et dans lequel les deux autres lasers sont agencés de manière coulissante en direction longitudinale de la table de support, et projettent, lorsqu'ils sont accouplés, une ligne laser commune (18) transversalement par rapport à la direction longitudinale de la table de support.

**13.** Dispositif selon l'une quelconque des revendications qui précèdent, **caractérisé en ce que** le dispositif est installé dans une autre pièce qu'un dispositif de tomographie (12) utilisé pour déterminer le modèle tridimensionnel virtuel du corps du patient (10) .

**14.** Procédé de marquage d'un champ de rayonnement produit à l'aide d'un modèle tridimensionnel virtuel du corps d'un patient sur la surface du corps du patient,
dans lequel les coordonnées d'au moins deux points de référence (26) sur la surface du corps du patient (10) sont déterminées dans un système de coordonnées local associé au dispositif,
dans lequel, à partir des coordonnées des points de référence (26) dans le système de coordonnées local et à partir de coordonnées des points de référence (26) dans un système de coordonnées virtuel du modèle tridimensionnel virtuel du corps du patient (10), une matrice de transformation est déterminée pour la transformation de coordonnées quelconques du système de coordonnées virtuel vers le système de coordonnées local,
et **caractérisé en ce que**, à l'aide de la matrice de transformation, des coordonnées sont transformées du système de coordonnées virtuel vers le système de coordonnées local et sont mises à disposition pour le marquage, et

dans lequel les points de coordonnées transformés sont parcourus en séquence à l'aide d'au moins un rayon laser.

15. Procédé selon la revendication 14, **caractérisé en ce que**, à l'aide de la matrice de transformation, les coordonnées au moins de l'isocentre de rayonnement sont transformées du système de coordonnées virtuel vers le système de coordonnées local et sont mises à disposition pour le marquage.

16. Procédé selon la revendication 14 ou selon la revendication 15, **caractérisé en ce que**, à l'aide de la matrice de transformation, les coordonnées au moins du contour du champ de rayonnement sont transformées du système de coordonnées virtuel vers le système de coordonnées local et sont mises à disposition pour le marquage.

17. Procédé selon l'une des revendications 14 à 16, **caractérisé en ce que** le système laser comporte au moins deux lasers qui génèrent chacun une ligne laser (28).

18. Procédé selon la revendication 17, **caractérisé en ce que** les coordonnées locales des points de référence (26) sont établies par une détermination de la translation des lignes laser (28) depuis le point zéro du système de coordonnées local vers les points de référence (26).

19. Procédé selon l'une des revendications 14 à 16, **caractérisé en ce que** les points de référence (26) peuvent être déterminés par une intersection de deux rayons laser (34) générés par au moins deux dispositifs de projection laser (32) sur la surface du patient.

20. Procédé selon la revendication 19, **caractérisé en ce que** les coordonnées transformées sont projetées sur la surface tridimensionnelle du corps du patient (10) en déplaçant au moins un rayon laser (34) généré par au moins un des dispositifs de projection laser (32) suffisamment vite le long des points de coordonnées transformés sur la surface du corps du patient (10), pour que l'impression d'un contour fermé soit obtenue sur la surface.

21. Procédé selon l'une des revendications 14 à 20, **caractérisé en ce que** les points de référence (26) sont marqués sur la peau du corps du patient (10) lors de la production du modèle tridimensionnel virtuel du corps du patient (10).

22. Procédé selon l'une des revendications 14 à 20, **caractérisé en ce qu'**un modèle tridimensionnel local du corps du patient (10) est produit dans le système de coordonnées local à l'aide d'un système de détection de surface.

23. Procédé selon l'une des revendications 14 à 20 ou selon la revendication 22, **caractérisé en ce qu'**une ligne laser (18) est tracée successivement sur la surface du corps du patient (10) à l'aide d'au moins un laser, **en ce que** la lumière laser réfléchie par le corps du patient (10) est détectée à l'aide d'au moins un dispositif de détection (20), et **en ce qu'**un modèle tridimensionnel local du corps du patient (10) est produit dans le système de coordonnées local à partir de la lumière laser détectée.

24. Procédé selon la revendication 23, **caractérisé en ce que** la matrice de transformation est fournie à partir d'une comparaison du modèle tridimensionnel local du corps du patient (10) avec le modèle tridimensionnel virtuel du corps du patient (10).

25. Procédé selon l'une des revendications 14 à 24, **caractérisé en ce qu'**au moins cinq lasers (14) qui génèrent chacun une ligne laser sont pourvus, dans lequel deux lasers (14) réglables en hauteur sont pourvus sur le côté d'une table de support pour le patient, qui projettent chacun une ligne horizontale le long de la table de support, et **en ce que** les autres lasers sont agencés au-dessus de la table de support, dans lequel un laser est agencé de manière coulissante transversalement par rapport à la direction longitudinale de la table de support et projette une ligne laser en direction longitudinale de la table de support, et dans lequel les deux autres lasers sont agencés de manière coulissante en direction longitudinale de la table de support, et projettent, lorsqu'ils sont accouplés, une ligne laser commune (18) transversalement par rapport à la direction longitudinale de la table de support.

26. Procédé selon l'une des revendications 14 à 25, **caractérisé en ce que** le procédé est mis en oeuvre dans une autre pièce que la détermination du modèle tridimensionnel virtuel du corps du patient (10).

FIG.1

FIG.2

FIG.3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4421315 A1
- DE 19524951 A1

- EP 1640922 A2
- DE 102008012496